# EUROPÄISCHE PATENTSCHRIFT

(11) **EP 3 412 208 B1**
(45) Veröffentlichungstag und Bekanntmachung des Hinweises auf die Patenterteilung: **26.05.2021**
(21) Anmeldenummer: 18153904.0
(22) Anmeldetag: 29.01.2018
(51) Int. Cl.: A61B 6/00, A61B 6/03, A61B 6/04

(54) **BEREITSTELLEN EINES MEDIZINISCHEN BILDES**
PROVISION OF A MEDICAL IMAGE
FOURNITURE D'UNE IMAGE MÉDICALE

(43) Veröffentlichungstag der Anmeldung: 12.12.2018
(73) Patentinhaber: Siemens Healthcare GmbH, 91052 Erlangen (DE); Universität Zürich, 8006 Zürich (CH)
(72) Erfinder: Guckenberger, Matthias, 8708 Männedorf (CH); Hofmann, Christian, 91052 Erlangen (DE); Ritter, André, 91077 Neunkirchen am Brand (DE); Tanadini-Lang, Stephanie, 8907 Wettswil (CH)

(56) Entgegenhaltungen:
- EP-A1- 1 542 165
- EP-A2- 2 037 413
- US-A1- 2004 081 269
- US-A1- 2007 270 689

## Beschreibung

Die Erfindung betrifft ein Verfahren für ein Bereitstellen eines mittels eines Computertomographen erfassten medizinischen Bildes eines Patienten, eine Computertomographieanlage für ein Bereitstellen eines medizinischen Bildes eines Patienten und ein Computerprogrammprodukt.

In der medizinischen Bildgebung ist eine bildgebende Messsequenz, welche typischerweise in einer medizinischen Bildgebungsmodalität, insbesondere in einem Computertomographen, durchführbar ist, vorzugsweise derart optimiert, dass in erster Linie ein physiologischer Zustand eines Patienten in einem medizinischen Bild sichtbar sein kann. Aufgrund unterschiedlicher technischer Herausforderungen ist es üblicherweise nicht oder nur unter hohem Aufwand möglich, mehrere, insbesondere unterschiedliche, physiologische Zustände des Patienten in dem medizinischen Bild sichtbar zu machen, insbesondere wenn das medizinische Bild einen vergleichsweise großen Messbereich aufweist oder mehrere medizinische Bilder über den Messbereich aufgeteilt sind, welche jeweils die physiologischen Zustände aufweisen sollen.

Ein Problem kann daraus entstehen, dass erste physiologische Zustände, insbesondere gemäß einer Atmung des Patienten, und zweite physiologische Zustände, insbesondere gemäß einer Kontrastmittelanreicherung in dem Patienten und/oder gemäß einer Jod-Verteilung in dem Patienten, sich in der jeweiligen Dynamik unterscheiden. Insbesondere aufgrund der unterschiedlichen Dynamik kann die bildgebende Messsequenz typischerweise entweder im Hinblick auf das Erfassen der ersten physiologischen Zustände oder auf das Erfassen der zweiten physiologischen Zustände optimiert werden. Ferner ist üblicherweise ein maximales Gesichtsfeld der medizinischen Bildgebungsmodalität kleiner als der für das gleichzeitige Erfassen der ersten physiologischen Zustände und der zweiten physiologischen Zustände notwendige Messbereich der bildgebenden Messsequenz. Sprich die ersten physiologischen Zustände und die zweiten physiologischen Zustände aufweisende Projektionsdaten können typischerweise nacheinander, insbesondere nicht gleichzeitig, erfasst werden.

Aus der US 2007/0270689 A1 ist ein Verfahren bekannt, wobei ein mittels eines Computertomographen erfasstes atemkorreliertes Bild mit einem mittels eines Angiographiesystems erfassten funktionellen Bild kombiniert wird, um einen interventionellen Eingriff präzise und sicher durchführen zu können.

Der Erfindung liegt die Aufgabe zu Grunde, ein Verfahren für ein Bereitstellen eines mittels eines Computertomographen erfassten medizinischen Bildes eines Patienten, eine Computertomographieanlage für ein Bereitstellen eines medizinischen Bildes eines Patienten und ein Computerprogrammproduktanzugeben, wobei das medizinische Bild unterschiedliche physiologische Zustände oder Zustandskombinationen aufweisen kann.

Die Aufgabe wird durch die Merkmale der unabhängigen Ansprüche gelöst. Vorteilhafte Ausgestaltungen sind in den Unteransprüchen beschrieben.

Das erfindungsgemäße Verfahren für ein Bereitstellen eines mittels eines Computertomographen erfassten medizinischen Bildes eines Patienten weist folgende Schritte auf:
- Erfassen von ersten Projektionsdaten eines ersten Messbereichs mittels des Computertomographen, wobei aus den ersten Projektionsdaten zumindest ein atemkorreliertes Bild des Patienten gemäß einer Atmung des Patienten rekonstruiert wird,
- Erfassen von zweiten Projektionsdaten eines zweiten Messbereichs mittels des Computertomographen, wobei aus den zweiten Projektionsdaten zumindest ein funktionelles Bild des Patienten gemäß einer ersten Rekonstruktionsvorschrift rekonstruiert wird und wobei sich der erste Messbereich und der zweite Messbereich zumindest teilweise überlappen,
- Registrieren eines Referenzbildes mit dem zumindest einen atemkorrelierten Bild des Patienten, wobei ein Deformationsmodell erzeugt wird,
- Anwenden des Deformationsmodells auf das zumindest eine funktionelle Bild des Patienten,
- Kombinieren des deformierten zumindest einen funktionellen Bildes des Patienten mit dem zumindest einen atemkorrelierten Bild des Patienten, wobei das medizinische Bild des Patienten erzeugt wird, und
- Bereitstellen des medizinischen Bildes des Patienten, dadurch gekennzeichnet,
dass das Referenzbild gemäß einer zweiten Rekonstruktionsvorschrift aus den zweiten Projektionsdaten rekonstruiert wird, wobei die erste Rekonstruktionsvorschrift in Hinblick auf einen funktionellen Kontrast optimiert ist und wobei die zweite Rekonstruktionsvorschrift in Hinblick auf einen anatomischen Kontrast optimiert ist.

Das Erfassen der ersten Projektionsdaten und/oder der zweiten Projektionsdaten kann ein Durchführen einer bildgebenden Messsequenz in einer medizinischen Bildgebungsmodalität, insbesondere in dem Computertomographen, umfassen. Typischerweise ist der Patient während der bildgebenden Messsequenz in der medizinischen Bildgebungsmodalität beispielsweise auf einer Patientenliege gelagert und/oder bei der medizinischen Bildgebungsmodalität positioniert. Vorzugsweise können die ersten Projektionsdaten und/oder die zweiten Projektionsdaten mittels des Computertomographen erfasst werden. Der erste Messbereich wird vorzugsweise von den ersten Projektionsdaten, insbesondere bei einer Durchführung einer ersten bildgebenden Messsequenz, abgebildet und/oder beschrieben. Der zweite Messbereich wird vorzugsweise von den zweiten Projektionsdaten, insbesondere bei einer Durchführung einer zweiten bildgebenden Messsequenz, abgebildet und/oder beschrieben. Typischerweise wird die erste bildgebende Messsequenz vor der zweiten bildgebenden Messsequenz durchgeführt. Grundsätzlich kann die zweite bildgebende Messsequenz vor der ersten bildgebenden Messsequenz durchgeführt werden.

Die ersten Projektionsdaten und/oder die zweiten Projektionsdaten können beispielsweise Rohdaten des Computertomographen aufweisen. Das zumindest eine atemkorrelierte Bild und/oder das zumindest eine funktionelle Bild des Patienten werden typischerweise mittels eines iterativen Rekonstruktionsverfahrens und/oder mittels einer gefilterten Rückprojektion aus den ersten Projektionsdaten bzw. aus den zweiten Projektionsdaten rekonstruiert. Das zumindest eine atemkorrelierte Bild des Patienten und/oder das zumindest eine funktionelle Bild des Patienten weisen vorzugsweise jeweils eine Verteilung von Bildwerten, insbesondere eine Hounsfield-Unit-Verteilung (HU), auf.

Die Atmung des Patienten kann beispielsweise durch ein physiologisches Sensorsystem, insbesondere während der ersten bildgebenden Messsequenz, ermittelt werden. Das physiologische Sensorsystem kann beispielsweise einen Atemgurt und/oder eine Kamera aufweisen. Beispielsweise können mittels des Atemgurts oder der Kamera mehrere Atemzustände der Atmung des Patienten ermittelt werden. Vorzugsweise werden die mehreren Atemzustände den ersten Projektionsdaten und/oder dem zumindest einen atemkorrelierten Bild zugeordnet. Die mehreren Atemzustände sind typischerweise zyklisch und/oder weisen beispielsweise die Zustände Einatmen, mittleres Atmen und/oder Ausatmen auf. Alternativ oder zusätzlich kann die Atmung des Patienten, insbesondere mittels eines Bilderkennungsalgorithmus, aus dem zumindest einen atemkorrelierten Bild bestimmt werden. Das zumindest eine atemkorrelierte Bild weist vorzugsweise die Atmung, insbesondere die mehreren Atemzustände der Atmung, des Patienten auf.

Das zumindest eine funktionelle Bild weist beispielsweise mehrere Zustände des funktionellen Vorgangs, insbesondere einer Kontrastmittelanreicherung typischerweise nach Injektion eines Kontrastmittelbolus, im Patienten auf. Üblicherweise werden die mehreren Kontrastmittelzustände durch die Injektion des Kontrastmittelbolus in den Patienten sichtbar. Die mehreren Kontrastmittelzustände weisen beispielsweise die Zustände keine Kontrastmittelanreicherung, ansteigende Kontrastmittelanreicherung, maximale Kontrastmittelanreicherung und/oder absteigende Kontrastmittelanreicherung auf. Alternativ oder zusätzlich kann das zumindest eine funktionelle Bild derart aus den zweiten Projektionsdaten rekonstruiert werden, dass das zumindest eine funktionelle Bild ein Mischbild, ein monoenergetisches Bild, ein virtual-non-contrast-Bild, ein Bild mit einer Fett-Verteilung, ein Bild mit einer Jod-Verteilung und/oder ein Bild mit einer Eisen-Verteilung aufweist. Das Bild mit der Jod-Verteilung kann beispielsweise die Kontrastmittelanreicherung aufweisen.

Typischerweise können die ersten Projektionsdaten und/oder die zweiten Projektionsdaten in Messdatenblöcke unterteilt werden. Ein Messdatenblock kann beispielsweise diejenigen Projektionsdaten aufweisen, welche während eines Zeitintervalls erfasst worden sind. Typischerweise kann das Zeitintervall mit einer Zeitauflösung der bildgebenden Messsequenz korrelieren oder der Zeitauflösung der bildgebenden Messsequenz entsprechen. Alternativ oder zusätzlich kann der Messdatenblock diejenigen Projektionsdaten aufweisen, welche einen Teil, insbesondere einen räumlichen Abschnitt, des ersten Messbereichs und/oder des zweiten Messbereichs aufweisen und/oder abbilden. Beispielsweise kann der räumliche Abschnitt eine z-Position des ersten Messbereichs und/oder des zweiten Messbereichs und/oder eine Ausdehnung des ersten Messbereichs und/oder des zweiten Messbereichs entlang der Längsachse der Patientenliege aufweisen. Die Patientenliege ist typischerweise parallel zur z-Achse der medizinischen Bildgebungsmodalität. Die Messdatenblöcke können jeweils einen Zustand, insbesondere der Atmung und/oder der Kontrastmittelanreicherung, aufweisen.

Vorzugsweise sind der erste Messbereich und der zweite Messbereich deckungsgleich. Typischerweise sind der erste Messbereich und/oder der zweite Messbereich größer als ein maximales Gesichtsfeld der medizinischen Bildgebungsmodalität, insbesondere als ein maximales Gesichtsfeld des Computertomographen. Vorzugsweise beträgt eine Schnittmenge zwischen dem ersten Messbereich und dem zweiten Messbereich, insbesondere parallel zur Längsachse der Patientenliege, mehr als 50%, besonders bevorzugt mehr als 90%. Die Schnittmenge zwischen dem ersten Messbereich und dem zweiten Messbereich kann beispielsweise als gemeinsamer Messbereich bezeichnet werden. Der erste Messbereich und/oder der zweite Messbereich können ein Organ des Patienten, beispielsweise eine Leber und/oder eine Lunge und/oder ein Herz des Patienten, aufweisen.

Vorteilhafterweise können die erste bildgebende Messsequenz und die zweite bildgebende Messsequenz unterschiedliche Messparameter aufweisen. Beispielsweise ist eine Tischvorschubgeschwindigkeit der zweiten bildgebenden Messsequenz schneller als eine Tischvorschubgeschwindigkeit der ersten bildgebenden Messsequenz, insbesondere um einen Faktor 1,5 oder 2, besonders vorteilhafterweise um einen Faktor 5 schneller. Vorzugsweise sind die Messparameter der ersten bildgebenden Messsequenz in Hinblick auf ein zeitaufgelöstes volumetrisches Erfassen der ersten Projektionsdaten optimiert und/oder die Messparameter der zweiten bildgebenden Messsequenz sind in Hinblick auf das Erfassen der Injektion des Kontrastmittelbolus optimiert. Die erste bildgebende Messsequenz ist vorzugsweise derart ausgebildet, dass die ersten Projektionsdaten die Atmung des Patienten insbesondere an jeder z-Position des ersten Messbereichs aufweisen. Vorteilhafterweise weisen die ersten Projektionsdaten die gleichen Atemzustände an jeder z-Position des ersten Messbereichs auf. Typischerweise werden die ersten Projektionsdaten so lange erfasst, bis die ersten Projektionsdaten eine gleiche Anzahl an Atemzuständen, insbesondere die gleichen Atemzustände, an jeder z-Position des ersten Messbereichs aufweisen. In diesem Fall kann die erste Messsequenz eine Spiral-CT-Messsequenz und/oder einer sequentiellen CT-Messsequenz beschreiben.

Die Rekonstruktionsvorschrift weist insbesondere eine Gewichtung der Projektionsdaten, insbesondere der zweiten Projektionsdaten, auf. In Abhängigkeit der Gewichtung der Projektionsdaten variiert beispielsweise ein Kontrast des rekonstruierten Bildes, insbesondere des zumindest einen funktionellen Bildes und/oder des Referenzbildes. Der Kontrast hängt typischerweise von der Verteilung an Bildwerten ab. Vorzugsweise können gemäß der ersten Rekonstruktionsvorschrift und der zweiten Rekonstruktionsvorschrift jeweils, insbesondere im Hinblick auf den Kontrast, unterschiedliche Bilder und/oder unterschiedliche Verteilungen an Bildwerten rekonstruiert werden.

Das Deformationsfeld kann insbesondere ein Vektorfeld aufweisen, welches eine Veränderung zwischen zwei Registrierungsbildern, insbesondere zwischen einem Startbild und einem Zielbild, abbildet. Beispielsweise weisen die zwei Registrierungsbilder das zumindest eine atemkorrelierte Bild und das Referenzbild auf. Das Deformationsfeld kann mehrere Vektorfelder aufweisen, welche einen Pfad von dem Startbild zum Zielbild beschreiben. Durch das Anwenden des Deformationsfelds werden die Projektionsdaten, insbesondere das zumindest eine atemkorrelierte Bild und/oder das Referenzbild, derart verformt, dass das Startbild vorzugsweise dem Zielbild, insbesondere im Hinblick auf Konturen, gleicht. Die Konturen können beispielsweise das Organ und/oder das Gewebe und/oder Gefäße und/oder Knochenmaterial und/oder einen Tumor beschreiben. Vorzugsweise kann durch Inversion des Deformationsfelds die Verformung umgedreht werden. Typischerweise sind das zumindest eine atemkorrelierte Bild und das Referenzbild jeweils das Startbild bzw. das Zielbild oder umgekehrt. Sprich je nach Ausführung kann das Deformationsfeld typischerweise ausgehend von dem zumindest einen atemkorrelierten Bild oder ausgehend von dem zumindest einen funktionellen Bild erzeugt und entsprechend angewendet werden.

Wenn das Referenzbild gemäß einer zweiten Rekonstruktionsvorschrift aus den zweiten Projektionsdaten rekonstruiert wird, wird insbesondere ein zu dem zumindest einen funktionellen Bild verschiedenes Bild aus den zweiten Projektionsdaten rekonstruiert. Die zweite Rekonstruktionsvorschrift unterscheidet sich typischerweise derart von der ersten Rekonstruktionsvorschrift, dass bei der jeweiligen Anwendung auf die zweiten Projektionsdaten unterschiedliche, insbesondere funktionelle, Bilder rekonstruiert werden. Vorzugsweise ist die zweite Rekonstruktionsvorschrift derart optimiert, dass das Registrieren des Referenzbildes gemäß der zweiten Rekonstruktionsvorschrift bessere Ergebnisse, insbesondere ein exakteres Deformationsmodell, liefert als das Registrieren des Referenzbildes gemäß der ersten Rekonstruktionsvorschrift.

Grundsätzlich kann das Registrieren ein Minimieren eines bildwertbasierten Abstandsmaßes umfassen. Das bildwertbasierte Abstandsmaß kann beispielsweise eine Summe oder einen Mittelwert von punktweise gebildeten quadratischen Differenzen zwischen dem Referenzbild, insbesondere dessen Verteilung an Bildwerten, und dem zumindest einen atemkorrelierten Bild, insbesondere dessen Verteilung an Bildwerten, des Patienten beschreiben. Das bildwertbasierte Abstandsmaß kann beispielsweise aufgrund einer systemischen Veränderung im Patienten, beispielsweise der Kontrastmittelanreicherung, ungeeignet sein. In diesem Fall kann beispielsweise ein local-crosscorrelation- und/oder ein mutual-information-Abstandsmaß verwendet werden.

Das Anwenden des Deformationsmodell umfasst insbesondere das Verformen des zumindest einen funktionellen Bildes derart, dass Konturen des zumindest einen funktionellen Bildes den Konturen des zumindest einen atemkorrelierten Bildes gleichen. Wenn das Referenzbild gemäß einer zweiten Rekonstruktionsvorschrift aus den zweiten Projektionsdaten rekonstruiert wird, wird vorteilhafterweise das Deformationsmodell bei ersten Registrierungsbildern ermittelt und das Deformationsmodell bei zweiten Registrierungsbildern angewendet, wobei die ersten Registrierungsbilder und die zweiten Registrierungsbilder typischerweise lediglich ein einziges Bild, insbesondere das Zielbild, also typischerweise das zumindest eine atemkorrelierte Bild, jeweils gemein haben.

Vorzugsweise umfasst das Kombinieren ein Verrechnen des deformierten zumindest einen funktionellen Bildes des Patienten mit dem zumindest einen atemkorrelierten Bild des Patienten derart, dass das medizinische Bild Bildinformation des deformierten zumindest einen funktionellen Bildes und Bildinformation des zumindest einen atemkorrelierten Bildes, beispielsweise verstärkt und/oder differenziert, aufweist. In diesem Fall geht typischerweise das medizinische Bild als einziges Bild aus der Kombination des zumindest einen funktionellen Bildes und des zumindest einen atemkorrelierten Bildes hervor. Alternativ oder zusätzlich kann das Kombinieren derart erfolgen, dass das medizinische Bild einen medizinischen Bilddatensatz aufweist, wobei der medizinische Bilddatensatz das zumindest eine funktionelle Bild und das zumindest eine atemkorrelierte Bild umfasst. In anderen Worten sind das zumindest eine funktionelle Bild und das zumindest eine atemkorrelierte Bild in einem einzelnen medizinischen Bilddatensatz, typischerweise allerdings als separate Bilder, enthalten. Es ist denkbar, dass der medizinische Bilddatensatz die separaten Bilder und ein kombiniertes medizinisches Bild aufweist.

Das Bereitstellen des medizinischen Bildes kann ein Übertragen des medizinischen Bildes von dem Computertomographen in ein Radiologieinformationssystem und/oder ein PACS-Bildarchivierungssystem umfassen. Alternativ oder zusätzlich kann das medizinische Bild, insbesondere dem Nutzer, auf einem Monitor angezeigt werden. Vorzugsweise ist der Monitor, beispielsweise zu dem Abrufen des medizinischen Bildes aus dem Radiologieinformationssystem bzw. dem PACS-Bildarchivierungssystem und/oder von der medizinischen Bildgebungsmodalität ausgebildet, und/oder zu einem Anzeigen des medizinischen Bildes ausgebildet. Grundsätzlich ist es denkbar, dass das medizinische Bild über ein Netzwerk, welches beispielsweise das Radiologieinformationssystem und/oder das PACS-Bildarchivierungssystem und/oder die medizinische Bildgebungsmodalität verbindet, ausgetauscht und beispielsweise auf einem Server gespeichert bzw. abgerufen werden kann. Zusätzlich zu dem medizinischen Bild können beispielsweise die ersten Projektionsdaten und/oder die zweiten Projektionsdaten bereitgestellt, insbesondere in das Radiologieinformationssystem und/oder das PACS-Bildarchivierungssystem übertragen und/oder auf dem Monitor angezeigt werden.

Das erfindungsgemäße Verfahren für das Bereitstellen des medizinischen Bildes des Patienten bringt insbesondere folgende Vorteile mit sich:
Das Erzeugen des medizinischen Bildes, welches vorzugsweise die Bildinformation von gemäß zwei separat durchgeführten bildgebenden Messsequenzen rekonstruierten Bildern aufweist, ist insbesondere für einen Nutzer, insbesondere einen Arzt, vorteilhaft, wenn der Arzt beispielsweise eine Bestrahlungsplanung für eine Krebstherapie des Patienten durchführt. Insbesondere wenn die Bestrahlungsplanung ein Organ betrifft, welches aufgrund der Positionierung aufgrund von Atmung oder Herzschlag relativ zum Thorax des Patienten beweglich ist, kann das medizinische Bild ermöglichen basierend auf dem zumindest einen atemkorrelierten Bild die Bewegung des Organs und basierend auf dem zumindest einen funktionellen Bild den funktionellen Vorgang, insbesondere eine Nekrose und/oder eine Durchblutung und/oder eine Kappilarisierung innerhalb des Organs, abzuschätzen. Typischerweise kann das medizinische Bild vorteilhafterweise die Jod-Verteilung im Patienten beim Atemzustand Einatmen aufweisen und/oder beispielsweise die Jod-Verteilung im Patienten beim Atemzustand Ausatmen aufweisen.

Das Erzeugen des medizinischen Bildes ist weiterhin in Hinblick auf eine Reduzierung von einer Untersuchungsdauer des Patienten vorteilhaft. Typischerweise korreliert die Untersuchungsdauer des Patienten beim Durchführen der bildgebenden Messsequenz in dem Computertomographen mit potentiell für den Patienten schädlichen Röntgenstrahlen, insbesondere einer Röntgendosis. Durch das Kombinieren der ersten Projektionsdaten, insbesondere des zumindest einen atemkorrelierten Bildes, und der zweiten Projektionsdaten, insbesondere des zumindest einen funktionellen Bildes, kann die Untersuchungsdauer und/oder vorzugsweise die mit der Röntgenstrahlung korrespondierende Röntgendosis verringert werden.

Wenn das Referenzbild gemäß der zweiten Rekonstruktionsvorschrift aus den zweiten Projektionsdaten rekonstruiert wird, ist vorzugsweise möglich basierend auf den zweiten Projektionsdaten ein in Hinblick auf die Registrierung optimales Bild gemäß der zweiten Rekonstruktionsvorschrift zu rekonstruieren und das daraus erzeugte Deformationsmodell beispielsweise auf das zumindest eine funktionelle Bild anzuwenden.

Eine Ausführungsform sieht vor, dass das deformierte zumindest eine funktionelle Bild des Patienten derart mit dem zumindest einen atemkorrelierten Bild des Patienten kombiniert wird, dass das medizinische Bild des Patienten ein Subtraktionsbild des Patienten aufweist, welches die Kontrastmittelanreicherung des Patienten aufweist. Vorzugsweise weist das Subtraktionsbild lediglich die Kontrastmittelanreicherung des Patienten auf. In anderen Worten wird insbesondere nicht mit Kontrastmittel angereichertes Gewebe oder Knochenmaterial durch die Subtraktion aus dem medizinischen Bild herausgerechnet. Grundsätzlich ist es denkbar, dass das Subtraktionsbild das Bild der Jod-Verteilung, das Bild der Fett-Verteilung und/oder das Bild der Eisen-Verteilung aufweist. Beispielsweise wird das deformierte zumindest eine funktionelle Bild des Patienten und das zumindest eine atemkorrelierte Bild des Patienten derart verrechnet, dass das zumindest eine atemkorrelierte Bild des Patienten von dem deformierten zumindest einen funktionellen Bild des Patienten abgezogen wird. Typischerweise beschreibt die Differenz die Kontrastmittelanreicherung im Patienten vor und nach der Injektion des Kontrastmittelbolus. Das Subtraktionsbild wird insbesondere durch das Kombinieren eines Prä-Kontrast-Bildes und eines Post-Kontrast-Bildes erzeugt, wobei typischerweise das Prä-Kontrast-Bild Bildinformation vor der Kontrastmittelanreicherung und das Post-Kontrast-Bild Bildinformation nach der Kontrastmittelanreicherung in dem jeweiligen Messbereich aufweist.

Eine Ausführungsform sieht vor, dass nach dem Erfassen der ersten Projektionsdaten und vor dem Erfassen der zweiten Projektionsdaten der Kontrastmittelbolus in den Patienten injiziert wird. In diesem Fall kann das Prä-Kontrast-Bild nicht aus den zweiten Projektionsdaten, sondern typischerweise lediglich aus den ersten Projektionsdaten rekonstruiert werden. Vorzugsweise werden das Prä-Kontrast-Bild aus den ersten Projektionsdaten und das Post-Kontrast-Bild aus den zweiten Projektionsdaten rekonstruiert. Dies ist insbesondere vorteilhaft, weil das Erfassen der zweiten Projektionsdaten kürzer ausfallen kann, wodurch insbesondere die Röntgendosis für den Patienten verringert sein kann. Diese Ausführungsform grenzt sich daher insbesondere zu der digitalen Subtraktions-Angiographie ab. Vorteilhafterweise werden das Prä-Kontrast-Bild und das Post-Kontrast-Bild aus unterschiedlichen Projektionsdaten rekonstruiert, wodurch die erste bildgebende Messsequenz und die zweite bildgebende Messsequenz unabhängig voneinander parametrisiert sein können und/oder, insbesondere in Hinblick auf die Röntgendosis, optimiert werden können. Die digitale Subtraktions-Angiographie erfordert im Gegensatz dazu üblicherweise, dass das Prä-Kontrast-Bild und das Post-Kontrast-Bild aus denselben Projektionsdaten rekonstruiert werden.

Eine Ausführungsform sieht vor, dass die zweiten Projektionsdaten und das daraus rekonstruierte Referenzbild die mehreren Zustände der Kontrastmittelanreicherung in dem Patienten aufweisen und wobei das Referenzbild in Abhängigkeit von den mehreren Zuständen der Kontrastmittelanreicherung mit dem zumindest einen atemkorrelierten Bild des Patienten registriert wird. Wenn das Referenzbild in Abhängigkeit von den mehreren Zuständen der Kontrastmittelanreicherung mit dem zumindest einen atemkorrelierten Bild des Patienten registriert wird, wird vorzugsweise gemäß einer Reihung der Zustände der Kontrastmittelanreicherung das Referenzbild registriert. In anderen Worten wird vorzugsweise zunächst dasjenige Referenzbild, welches vorzugsweise dem zumindest einen atemkorrelierten Bild vergleichsweise am ähnlichsten ist, mit dem zumindest einen atemkorrelierten Bild registriert und so weiter. In Abhängigkeit von den mehreren Zuständen der Kontrastmittelanreicherung bedeutet insbesondere, dass beispielsweise die zweiten Projektionsdaten derart sortiert werden, dass die Kontrastmittelanreicherung ansteigt oder absteigt. Auch andere Reihungen sind denkbar. Vorzugsweise kann das Deformationsmodell, insbesondere die Vektorfelder, schrittweise gemäß dem Anstieg der Kontrastmittelanreicherung erzeugt und/oder auf das zumindest eine funktionelle Bild angewendet werden.

Eine Ausführungsform sieht vor, dass aus den ersten Projektionsdaten das zumindest eine atemkorrelierte Bild für mehrere Atemzustände der Atmung des Patienten rekonstruiert wird und wobei durch das Registrieren des Referenzbildes mit dem jeweiligen zumindest einen atemkorrelierten Bild für die mehreren Atemzustände der Atmung des Patienten mehrere Deformationsmodelle erzeugt werden. In anderen Worten können vorzugsweise die mehreren Deformationsmodelle in Abhängigkeit von den mehreren Atemzuständen erzeugt werden.

Eine Ausführungsform sieht vor, dass durch das Anwenden der mehreren Deformationsmodelle auf das zumindest eine funktionelle Bild des Patienten und durch das Kombinieren des deformierten zumindest einen funktionellen Bildes für die mehreren Atemzustände des Patienten mit dem zumindest einen atemkorrelierten Bild des Patienten das medizinische Bild für die mehreren Atemzustände der Atmung des Patienten erzeugt wird. Das zumindest eine atemkorrelierte Bild für mehrere Atemzustände der Atmung des Patienten weist daher beispielsweise Bildinformation beim Einatmen und Bildinformation beim Ausatmen auf. Die mehreren Deformationsmodelle ermöglichen in diesem Fall das Verformen des zumindest einen funktionellen Bildes derart, dass insbesondere die Konturen des deformierten zumindest einen funktionellen Bildes des Patienten jeweils an das Einatmen oder an das Ausatmen angepasst werden. Vorteilhafterweise weist das medizinische Bild für die mehreren Atemzustände der Atmung des Patienten das deformierte zumindest eine funktionelle Bild beispielsweise beim Einatmen und/oder beim Ausatmen auf.

Eine Ausführungsform sieht vor, dass die zweiten Projektionsdaten mittels einer bildgebenden spektralen Messsequenz in dem Computertomographen erfasst werden und einen ersten Energiebereich sowie einen zweiten Energiebereich aufweisen. In diesem Fall entspricht insbesondere die zweite bildgebende Messsequenz der bildgebenden spektralen Messsequenz. Beispielsweise unterscheiden sich der erste Energiebereich und der zweite Energiebereich in Hinblick auf eine Energie der Röntgenstrahlen. Üblicherweise ist die Bildinformation des zumindest einen atemkorrelierten Bildes und/oder die Bildinformation des zumindest einen funktionellen Bildes abhängig von dem ersten Energiebereich und/oder dem zweiten Energiebereich. Beispielsweise erzeugt ein Röntgenstrahler die Röntgenstrahlen verteilt über ein Röntgenspektrum in Abhängigkeit der angelegten Beschleunigungsspannung zwischen Kathode und Anode. Der erste Energiebereich und der zweite Energiebereich können sich beispielsweise in der Beschleunigungsspannung unterscheiden. Üblicherweise ist es möglich, die Beschleunigungsspannung innerhalb desselben Röntgenstrahlers zeitlich zu variieren und/oder zwei Röntgenstrahler zu verwenden, wobei in diesem Fall typischerweise beide Röntgenstrahler mit unterschiedlichen Beschleunigungsspannungen betrieben werden. Ein Computertomograph, welcher zwei Röntgenstrahler aufweist, wird typischerweise Dual-Source- und/oder Dual-Energy-Computertomograph genannt. Vorzugsweise können beide Röntgenstrahler gleichzeitig, insbesondere gemäß der zweiten bildgebenden Messsequenz, die Röntgenstrahlen emittieren. Alternativ oder zusätzlich werden die Röntgenstrahlen mittels eines energiesensitiven Röntgendetektors, beispielsweise eines Photon-Counting-Detektor, erfasst. Wenn die zweiten Projektionsdaten den ersten Energiebereich und den zweiten Energiebereich aufweisen, kann vorzugsweise aus den zweiten Projektionsdaten das Mischbild, insbesondere bei 120 keV, das monoenergetische Bild, das virtual-non-contrast-Bild, das Bild mit der Fett-Verteilung, das Bild mit der Jod-Verteilung und/oder das Bild mit der Eisen-Verteilung rekonstruiert werden.

Eine Ausführungsform sieht vor, dass das Referenzbild gemäß der zweiten Rekonstruktionsvorschrift aus den zweiten Projektionsdaten rekonstruiert wird, wobei die erste Rekonstruktionsvorschrift eine erste Mischung der zweiten Projektionsdaten gemäß dem ersten Energiebereich und dem zweiten Energiebereich beschreibt und wobei die zweite Rekonstruktionsvorschrift eine zweite Mischung der zweiten Projektionsdaten gemäß dem ersten Energiebereich und dem zweiten Energiebereich beschreibt. Die erste Mischung und/oder die zweite Mischung beschreiben typischerweise jeweils ein Kombinieren der ersten Projektionsdaten mit den zweiten Projektionsdaten und/oder ein Anwenden eines Multiband-Filters auf die ersten Projektionsdaten und/oder die zweiten Projektionsdaten mit unterschiedlichen Gewichtungen. Besonders vorteilhafterweise werden für diese Ausführungsform die zweiten Projektionsdaten in einem Dual-Source-Computertomographen und/oder mittels des Photon-Counting-Detektors erfasst. Typischerweise ist das Referenzbild gemäß der zweiten Rekonstruktionsvorschrift, beispielsweise das Mischbild und/oder das virtual-non-contrast-Bild, insbesondere bildwert-basiert, ähnlicher zum zumindest einen atemkorrelierten Bild als das zumindest eine funktionelle Bild gemäß der ersten Rekonstruktionsvorschrift, insbesondere das Bild mit der Fett-Verteilung, das Bild mit der Jod-Verteilung und/oder das Bild mit der Eisen-Verteilung.

Das Referenzbild wird gemäß der zweiten Rekonstruktionsvorschrift aus den zweiten Projektionsdaten rekonstruiert, wobei die erste Rekonstruktionsvorschrift in Hinblick auf einen funktionellen Kontrast optimiert ist und wobei die zweite Rekonstruktionsvorschrift in Hinblick auf einen anatomischen Kontrast optimiert ist. In anderen Worten ist die erste Rekonstruktionsvorschrift, insbesondere die erste Mischung, und die zweite Rekonstruktionsvorschrift, insbesondere die zweite Mischung, derart ausgebildet, dass vorzugsweise das Referenzbild gemäß der zweiten Rekonstruktionsvorschrift und das zumindest eine atemkorrelierte Bild eine Anatomie des Patienten aufweisen, wobei das zumindest eine funktionelle Bild gemäß der ersten Rekonstruktionsvorschrift vorzugsweise den funktionellen Vorgang, beispielsweise die Kontrastmittelanreicherung, aufweist. Vorteilhafterweise weist das Referenzbild den optimierten anatomischen Kontrast auf, wodurch typischerweise das Registrieren des Referenzbildes mit dem zumindest einen atemkorrelierten Bild und somit das Deformationsmodell verbessert wird. In anderen Worten kann vorteilhafterweise basierend auf den zweiten Projektionsdaten allein ein optimales Deformationsmodell berechnet werden, wobei das optimale Deformationsmodell vorteilhafterweise auf das zumindest eine funktionelle Bild angewendet wird, welches wiederum auf den zweiten Projektionsdaten basiert.

Die erfindungsgemäße Computertomographieanlage für ein Bereitstellen eines medizinischen Bildes eines Patienten weist den Computertomographen und eine Recheneinheit auf, wobei der Computertomograph zumindest einen Röntgenstrahler und zumindest einen Röntgendetektor aufweist. Der Computertomograph ist vorzugsweise zur Durchführung der ersten bildgebenden Messsequenz und/oder der zweiten bildgebenden Messsequenz ausgebildet. Die Recheneinheit kann beispielsweise mittels des Netzwerks mit dem Radiologieinformationssystem und/oder dem PACS-Bildarchivierungssystem zu dem Bereitstellen des medizinischen Bildes verbunden sein. Alternativ oder zusätzlich kann die Computertomographieanlage für das Bereitstellen des medizinischen Bildes des Patienten, insbesondere die Recheneinheit, den Monitor aufweisen. Die Recheneinheit ist vorzugsweise mit dem Computertomographen derart verbunden, dass die ersten Projektionsdaten, welche insbesondere bei der Durchführung der ersten bildgebenden Messsequenz erfasst werden, und/oder die zweiten Projektionsdaten, welche insbesondere bei der Durchführung der zweiten bildgebenden Messsequenz erfasst werden, von dem Computertomographen zur Recheneinheit übertragen werden können.

Eine Ausführungsform sieht vor, dass der Computertomograph zwei Röntgenstrahler und zwei Röntgendetektoren aufweist. In diesem Fall kann der Computertomograph Dual-Source-Computertomograph und/oder Dual-Energy-Computertomograph genannt werden.

Das erfindungsgemäße Computerprogrammprodukt weist Programmcodemittel auf, welche in die Recheneinheit ladbar sind, um ein erfindungsgemäßes Verfahren für ein Bereitstellen eines medizinischen Bildes eines Patienten auszuführen, wenn die Programmcodemittel in der Recheneinheit ausgeführt werden.

Das Computerprogrammprodukt kann ein Computerprogramm sein oder ein Computerprogramm umfassen. Das Computerprogrammprodukt weist insbesondere die Programmcodemittel auf, welche die erfindungsgemäßen Verfahrensschritte abbilden. Dadurch kann das erfindungsgemäße Verfahren definiert und wiederholbar ausgeführt sowie eine Kontrolle über eine Weitergabe des erfindungsgemäßen Verfahrens ausgeübt werden. Das Computerprogrammprodukt ist vorzugsweise derart konfiguriert, dass die Recheneinheit mittels des Computerprogrammprodukts die erfindungsgemäßen Verfahrensschritte ausführen kann. Die Programmcodemittel können insbesondere in einen Speicher der Recheneinheit geladen und typischerweise mittels eines Prozessors der Recheneinheit mit Zugriff auf den Speicher ausgeführt werden. Wenn das Computerprogrammprodukt, insbesondere die Programmcodemittel, in der Recheneinheit ausgeführt wird, können typischerweise alle erfindungsgemäßen Ausführungsformen des beschriebenen Verfahrens durchgeführt werden. Das Computerprogrammprodukt ist beispielsweise auf einem physischen, computerlesbaren Medium gespeichert und/oder digital als Datenpaket in einem Computernetzwerk hinterlegt. Das Computerprogrammprodukt kann das physische, computerlesbare Medium und/oder das Datenpaket in dem Computernetzwerk darstellen. So kann die Erfindung auch von dem physischen, computerlesbaren Medium und/oder dem Datenpaket in dem Computernetzwerk ausgehen. Das physische, computerlesbare Medium ist üblicherweise unmittelbar mit der Recheneinheit verbindbar, beispielsweise indem das physische, computerlesbare Medium in ein DVD-Laufwerk eingelegt oder in einen USB-Port gesteckt wird, wodurch die Recheneinheit auf das physische, computerlesbare Medium insbesondere lesend zugreifen kann. Das Datenpaket kann vorzugsweise aus dem Computernetzwerk abgerufen werden. Das Computernetzwerk kann die Recheneinheit aufweisen oder mittels einer Wide-Area-Network- (WAN) bzw. einer (Wireless-)Local-Area-Network-Verbindung (WLAN oder LAN) mit der Recheneinheit mittelbar verbunden sein. Beispielsweise kann das Computerprogrammprodukt digital auf einem Cloud-Server an einem Speicherort des Computernetzwerks hinterlegt sein, mittels des WAN über das Internet und/oder mittels des WLAN bzw. LAN auf die Recheneinheit insbesondere durch das Aufrufen eines Downloadlinks, welcher zu dem Speicherort des Computerprogrammprodukts verweist, übertragen werden.

Im Folgenden wird die Erfindung anhand der in den Figuren dargestellten Ausführungsbeispiele näher beschrieben und erläutert. Grundsätzlich werden in der folgenden Figurenbeschreibung im Wesentlichen gleich bleibende Strukturen und Einheiten mit demselben Bezugszeichen wie beim erstmaligen Auftreten der jeweiligen Struktur oder Einheit benannt.

Es zeigen:
Fig. 1 ein Flussdiagramm eines Verfahrens für ein Bereitstellen eines mittels eines Computertomographen erfassten medizinischen Bildes eines Patienten in einem ersten Ausführungsbeispiel,
Fig. 2 eine Computertomographieanlage für ein Bereitstellen eines medizinischen Bildes eines Patienten,
Fig. 3 ein Flussdiagramm eines Verfahrens für ein Bereitstellen eines mittels eines Computertomographen erfassten medizinischen Bildes eines Patienten in einem zweiten Ausführungsbeispiel.

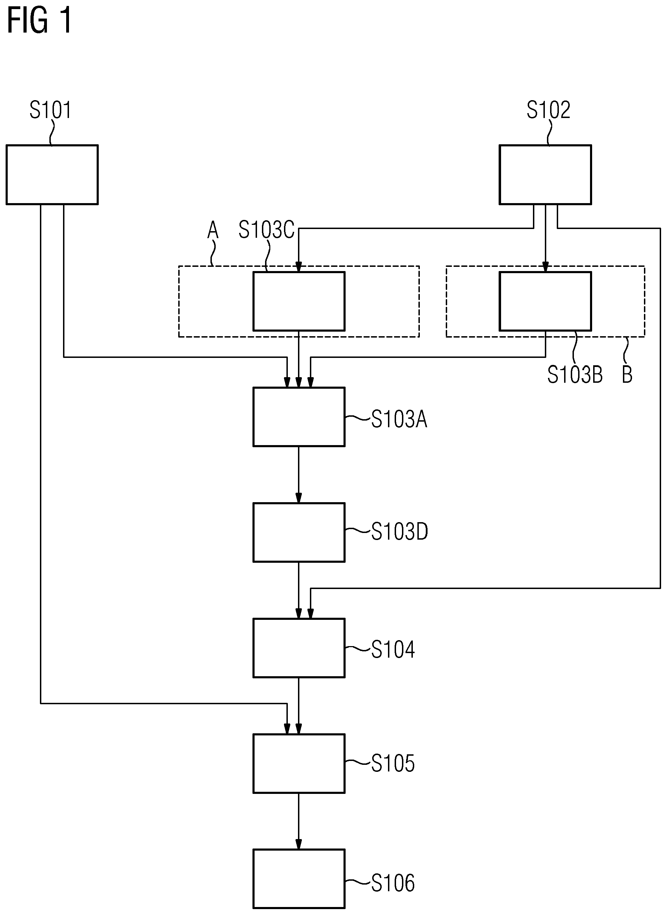

**Fig. 1** zeigt ein Flussdiagramm eines Verfahrens für ein Bereitstellen eines mittels eines Computertomographen erfassten medizinischen Bildes eines Patienten in einem ersten Ausführungsbeispiel.

Verfahrensschritt S101 kennzeichnet das Erfassen von ersten Projektionsdaten eines ersten Messbereichs mittels des Computertomographen, wobei aus den ersten Projektionsdaten zumindest ein atemkorreliertes Bild des Patienten gemäß einer Atmung des Patienten rekonstruiert wird.

Verfahrensschritt S102 kennzeichnet das Erfassen von zweiten Projektionsdaten eines zweiten Messbereichs mittels des Computertomographen, wobei aus den zweiten Projektionsdaten zumindest ein funktionelles Bild des Patienten gemäß einer ersten Rekonstruktionsvorschrift rekonstruiert wird und wobei sich der erste Messbereich und der zweite Messbereich zumindest teilweise überlappen.

Verfahrensschritt S103A kennzeichnet das Registrieren eines Referenzbildes mit dem zumindest einen atemkorrelierten Bild des Patienten.

Verfahrensschritt S103B und Verfahrensschritt S103C bilden zwei Alternativen A und B. In anderen Worten wird entweder Verfahrensschritt S103B oder Verfahrensschritt S103C durchgeführt. Sprich Verfahrensschritt S103B und Verfahrensschritt S103C bieten zwei unabhängige Möglichkeiten A, B das Referenzbild zu erhalten.

Verfahrensschritt S103B kennzeichnet, dass das Referenzbild dem zumindest einen funktionellen Bild des Patienten entspricht.

Verfahrensschritt S103C kennzeichnet, dass das Referenzbild gemäß einer zweiten Rekonstruktionsvorschrift aus den zweiten Projektionsdaten rekonstruiert wird.

Verfahrensschritt S103D kennzeichnet, dass ein Deformationsmodell erzeugt wird.

Verfahrensschritt S104 kennzeichnet das Anwenden des Deformationsmodells auf das zumindest eine funktionelle Bild des Patienten.

Verfahrensschritt S105 kennzeichnet das Kombinieren des deformierten zumindest einen funktionellen Bildes des Patienten mit dem zumindest einen atemkorrelierten Bild des Patienten, wobei das medizinische Bild des Patienten erzeugt wird.

Verfahrensschritt S106 kennzeichnet das Bereitstellen des medizinischen Bildes des Patienten.

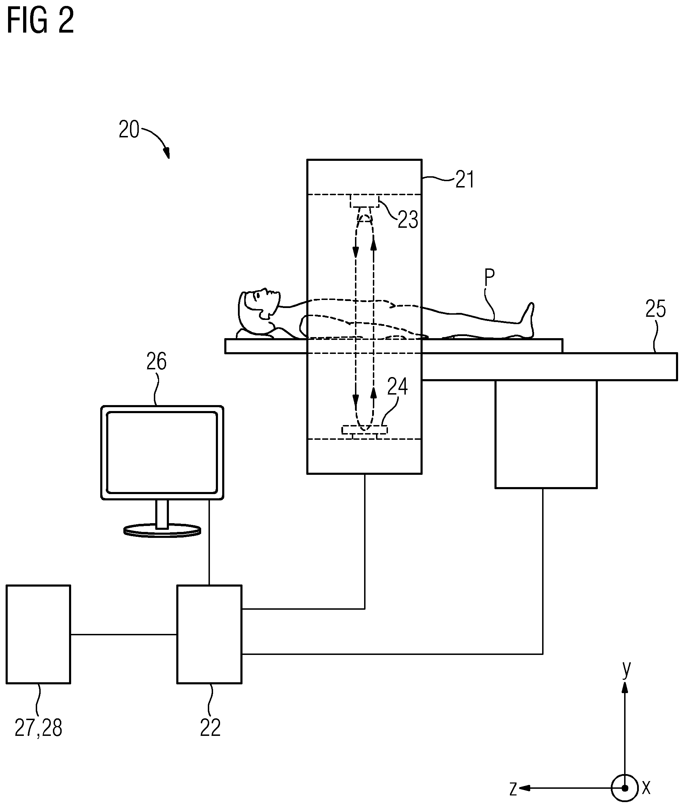

**Fig. 2** zeigt eine Computertomographieanlage 20 für ein Bereitstellen eines medizinischen Bildes eines Patienten P, wobei die Computertomographieanlage 20 einen Computertomographen 21 und eine Recheneinheit 22 aufweist. Der Computertomograph 21 weist zumindest einen Röntgenstrahler 23 und zumindest einen Röntgendetektor 24 auf, wobei der zumindest eine Röntgenstrahler 23 und der zumindest eine Röntgendetektor 24 um eine Patientenliege 25 drehbar angeordnet sind. Der Patient P ist auf der Patientenliege 25 gelagert, wobei die Patientenliege 25 parallel zur Raumachse z ausgebildet ist. Die Recheneinheit 22 weist einen Monitor 26 auf, welcher beispielsweise eine graphische Benutzeroberfläche sowie Eingabemittel aufweist. Beispielsweise kann der Nutzer und/oder der Arzt mittels der graphischen Benutzeroberfläche Messparameter einer ersten bildgebenden Messsequenz und/oder einer zweiten bildgebenden Messsequenz festlegen. Die Recheneinheit 22 ist mit einem Radiologieinformationssystem 27 und einem PACS-Bildarchivierungssystem 28 verbunden.

Grundsätzlich ist es denkbar, dass der Computertomograph 21 zwei Röntgenstrahler und zwei Röntgendetektoren aufweist, wobei vorzugsweise beide Röntgenstrahler unterschiedliche Beschleunigungsspannungen, insbesondere während der zweiten bildgebenden Messsequenz, aufweisen. Vorzugsweise weisen die zweiten Projektionsdaten in diesem Fall einen ersten Energiebereich und einen zweiten Energiebereich auf.

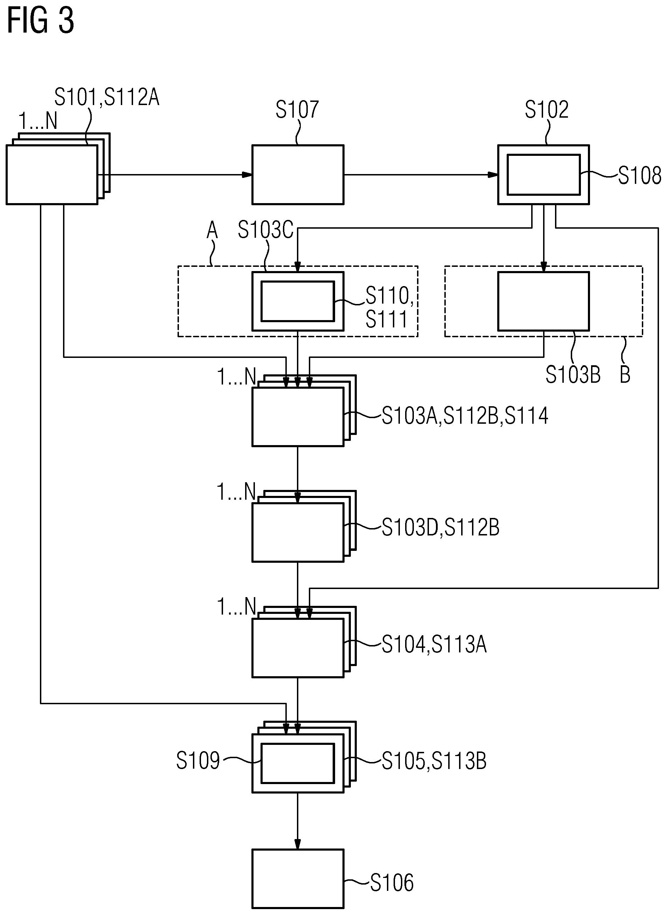

**Fig. 3** zeigt ein Flussdiagramm eines Verfahrens für ein Bereitstellen eines mittels eines Computertomographen erfassten medizinischen Bildes eines Patienten in einem zweiten Ausführungsbeispiel. Die in Fig. 3 gezeigten Verfahrensschritte S107 bis S114 können quasi beliebig kombiniert werden.

Verfahrensschritt S107 kennzeichnet, dass nach dem Erfassen der ersten Projektionsdaten und vor dem Erfassen der zweiten Projektionsdaten ein Kontrastmittelbolus in den Patienten injiziert wird.

Verfahrensschritt S108 kennzeichnet, dass die zweiten Projektionsdaten mittels einer bildgebenden spektralen Messsequenz in dem Computertomographen erfasst werden und einen ersten Energiebereich sowie einen zweiten Energiebereich aufweisen.

Verfahrensschritt S110 kennzeichnet, dass das Referenzbild gemäß der zweiten Rekonstruktionsvorschrift aus den zweiten Projektionsdaten rekonstruiert wird, wobei die erste Rekonstruktionsvorschrift eine erste Mischung der zweiten Projektionsdaten gemäß dem ersten Energiebereich und dem zweiten Energiebereich beschreibt und wobei die zweite Rekonstruktionsvorschrift eine zweite Mischung der zweiten Projektionsdaten gemäß dem ersten Energiebereich und dem zweiten Energiebereich beschreibt.

Verfahrensschritt S111 kennzeichnet, dass das Referenzbild gemäß der zweiten Rekonstruktionsvorschrift aus den zweiten Projektionsdaten rekonstruiert wird, wobei die erste Rekonstruktionsvorschrift in Hinblick auf einen funktionellen Kontrast optimiert ist und wobei die zweite Rekonstruktionsvorschrift in Hinblick auf einen anatomischen Kontrast optimiert ist.

Verfahrensschritt S112A kennzeichnet, dass aus den ersten Projektionsdaten das zumindest eine atemkorrelierte Bild für mehrere Atemzustände der Atmung des Patienten rekonstruiert wird.

Verfahrensschritt S112B kennzeichnet, dass durch das Registrieren des Referenzbildes mit dem jeweiligen zumindest einen atemkorrelierten Bild für die mehreren Atemzustände der Atmung des Patienten mehrere Deformationsmodelle erzeugt werden.

Verfahrensschritt S113A kennzeichnet, dass die mehreren Deformationsmodelle auf das zumindest eine funktionelle Bild des Patienten angewendet werden.

Verfahrensschritt S113B kennzeichnet, dass durch das Kombinieren des deformierten zumindest einen funktionellen Bildes für die mehreren Atemzustände des Patienten mit dem zumindest einen atemkorrelierten Bild des Patienten das medizinische Bild für die mehreren Atemzustände der Atmung des Patienten erzeugt wird. Das medizinische Bild kann einen medizinischen Bilddatensatz aufweisen, wobei der medizinische Bilddatensatz separate Bilder für die jeweiligen Atemzustände der Atmung des Patienten aufweist.

Verfahrensschritt S114 kennzeichnet, dass die zweiten Projektionsdaten und das daraus rekonstruierte Referenzbild mehrere Zustände einer Kontrastmittelanreicherung in dem Patienten aufweisen, wobei das Referenzbild in Abhängigkeit von den mehreren Zuständen der Kontrastmittelanreicherung mit dem zumindest einen atemkorrelierten Bild des Patienten registriert wird.

Verfahrensschritt S109 kennzeichnet, dass das deformierte zumindest eine funktionelle Bild des Patienten derart mit dem zumindest einen atemkorrelierten Bild des Patienten kombiniert wird, dass das medizinische Bild des Patienten ein Subtraktionsbild des Patienten aufweist, welches eine Kontrastmittelanreicherung des Patienten aufweist.

Obwohl die Erfindung im Detail durch die bevorzugten Ausführungsbeispiele näher illustriert und beschrieben wurde, so ist die Erfindung dennoch nicht durch die offenbarten Beispiele eingeschränkt und andere Variationen können vom Fachmann hieraus abgeleitet werden, ohne den Schutzumfang der Erfindung zu verlassen.

## Patentansprüche

1. Verfahren für ein Bereitstellen eines mittels eines Computertomographen (21) erfassten medizinischen Bildes eines Patienten (P), mit den folgenden Schritten:
- Erfassen von ersten Projektionsdaten eines ersten Messbereichs mittels des Computertomographen (21), wobei aus den ersten Projektionsdaten zumindest ein atemkorreliertes Bild des Patienten gemäß einer Atmung des Patienten rekonstruiert wird,
- Erfassen von zweiten Projektionsdaten eines zweiten Messbereichs mittels des Computertomographen (21), wobei aus den zweiten Projektionsdaten zumindest ein funktionelles Bild des Patienten gemäß einer ersten Rekonstruktionsvorschrift rekonstruiert wird und wobei sich der erste Messbereich und der zweite Messbereich zumindest teilweise überlappen,
- Registrieren eines Referenzbildes mit dem zumindest einen atemkorrelierten Bild des Patienten, wobei ein Deformationsmodell erzeugt wird,
- Anwenden des Deformationsmodells auf das zumindest eine funktionelle Bild des Patienten,
- Kombinieren des deformierten zumindest einen funktionellen Bildes des Patienten mit dem zumindest einen atemkorrelierten Bild des Patienten, wobei das medizinische Bild des Patienten erzeugt wird, und
- Bereitstellen des medizinischen Bildes des Patienten,
**dadurch gekennzeichnet,**
**dass** das Referenzbild gemäß einer zweiten Rekonstruktionsvorschrift aus den zweiten Projektionsdaten rekonstruiert wird, wobei die erste Rekonstruktionsvorschrift in Hinblick auf einen funktionellen Kontrast optimiert ist und wobei die zweite Rekonstruktionsvorschrift in Hinblick auf einen anatomischen Kontrast optimiert ist.

2. Verfahren nach Anspruch 1, wobei das deformierte zumindest eine funktionelle Bild des Patienten derart mit dem zumindest einen atemkorrelierten Bild des Patienten kombiniert wird, dass das medizinische Bild des Patienten ein Subtraktionsbild des Patienten aufweist, welches eine Kontrastmittelanreicherung des Patienten aufweist.

3. Verfahren nach einem der vorhergehenden Ansprüche, wobei nach dem Erfassen der ersten Projektionsdaten und vor dem Erfassen der zweiten Projektionsdaten ein Kontrastmittelbolus in den Patienten injiziert wird.

4. Verfahren nach einem der vorhergehenden Ansprüche, wobei die zweiten Projektionsdaten und das daraus rekonstruierte Referenzbild mehrere Zustände einer Kontrastmittelanreicherung in dem Patienten aufweisen und wobei das Referenzbild in Abhängigkeit von den mehreren Zuständen der Kontrastmittelanreicherung mit dem zumindest einen atemkorrelierten Bild des Patienten registriert wird.

5. Verfahren nach einem der vorhergehenden Ansprüche, wobei aus den ersten Projektionsdaten das zumindest eine atemkorrelierte Bild für mehrere Atemzustände der Atmung des Patienten rekonstruiert wird und wobei durch das Registrieren des Referenzbildes mit dem jeweiligen zumindest einen atemkorrelierten Bild für die mehreren Atemzustände der Atmung des Patienten mehrere Deformationsmodelle erzeugt werden.

6. Verfahren nach Anspruch 5, wobei durch das Anwenden der mehreren Deformationsmodelle auf das zumindest eine funktionelle Bild des Patienten und durch das Kombinieren des deformierten zumindest einen funktionellen Bildes für die mehreren Atemzustände des Patienten mit dem zumindest einen atemkorrelierten Bild des Patienten das medizinische Bild für die mehreren Atemzustände der Atmung des Patienten erzeugt wird.

7. Verfahren nach einem der vorhergehenden Ansprüche, wobei die zweiten Projektionsdaten mittels einer bildgebenden spektralen Messsequenz in dem Computertomographen (21) erfasst werden und einen ersten Energiebereich sowie einen zweiten Energiebereich aufweisen.

8. Verfahren nach Anspruch 7, wobei das Referenzbild gemäß der zweiten Rekonstruktionsvorschrift aus den zweiten Projektionsdaten rekonstruiert wird und wobei die erste Rekonstruktionsvorschrift eine erste Mischung der zweiten Projektionsdaten gemäß dem ersten Energiebereich und dem zweiten Energiebereich beschreibt und wobei die zweite Rekonstruktionsvorschrift eine zweite Mischung der zweiten Projektionsdaten gemäß dem ersten Energiebereich und dem zweiten Energiebereich beschreibt.

9. Computertomographieanlage (20) für ein Bereitstellen eines medizinischen Bildes eines Patienten (P), aufweisend
- einen Computertomographen (21), wobei der Computertomograph (21) zumindest einen Röntgenstrahler (23) und zumindest einen Röntgendetektor (24) aufweist, und
- eine Recheneinheit (22), wobei die Computertomographieanlage (20) zur Durchführung eines Verfahrens nach einem der Ansprüche 1 bis 8 ausgebildet ist.

10. Computertomographieanlage (20) nach Anspruch 9, wobei der Computertomograph (21) zwei Röntgenstrahler (23) und zwei Röntgendetektoren (24) aufweist.

11. Computerprogrammprodukt, aufweisend Programmcodemittel, welche in eine Recheneinheit (22) ladbar sind, um ein Verfahren nach einem der Ansprüche 1 bis 8 auszuführen, wenn die Programmcodemittel in der Recheneinheit (22) ausgeführt werden.

## Claims

1. Method for providing a medical image of a patient (P), which image has been acquired by means of a computed tomography apparatus (21), comprising the following steps:
- acquiring first projection data of a first measurement region by means of the computed tomography apparatus (21), wherein at least one respiration-correlated image of the patient corresponding to respiration of the patient is reconstructed from the first projection data;
- acquiring second projection data of a second measurement region by means of the computed tomography apparatus (21), wherein at least one functional image of the patient is reconstructed under a first reconstruction rule from the second projection data, and wherein the first measurement region and the second measurement region overlap at least partially;
- registering a reference image to the at least one respiration-correlated image of the patient, wherein a deformation model is produced;
- applying the deformation model to the at least one functional image of the patient;
- combining the deformed at least one functional image of the patient with the at least one respiration-correlated image of the patient, thereby producing the medical image of the patient; and
- providing the medical image of the patient,
**characterised in that**
the reference image is reconstructed under a second reconstruction rule from the second projection data, wherein the first reconstruction rule is optimised with regard to a functional contrast, and wherein the second reconstruction rule is optimised with regard to an anatomical contrast.

2. Method according to claim 1, wherein the deformed at least one functional image of the patient is combined with the at least one respiration-correlated image of the patient in such a way that the medical image of the patient has a subtraction image of the patient, which image has a contrast enhancement of the patient.

3. Method according to one of the preceding claims, wherein a contrast agent bolus is injected into the patient after the acquisition of the first projection data and before the acquisition of the second projection data.

4. Method according to one of the preceding claims, wherein the second projection data and the reference image reconstructed therefrom has a plurality of states of a contrast enhancement in the patient, and wherein the reference image is registered to the at least one respiration-correlated image of the patient on the basis of the plurality of states of the contrast enhancement.

5. Method according to one of the preceding claims, wherein the at least one respiration-correlated image for a plurality of respiratory states of the respiration of the patient is reconstructed from the first projection data, and wherein a plurality of deformation models are produced by registering the reference image to the corresponding at least one respiration-correlated image for the plurality of respiratory states of the respiration of the patient.

6. Method according to claim 5, wherein the medical image for the plurality of respiratory states of the respiration of the patient is produced by applying the plurality of deformation models to the at least one functional image of the patient and by combining the deformed at least one functional image for the plurality of respiratory states of the patient with the at least one respiration-correlated image of the patient.

7. Method according to one of the preceding claims, wherein the second projection data is acquired by means of an imaging spectral measurement sequence in the computed tomography apparatus (21), and has a first energy band and a second energy band.

8. Method according to claim 7, wherein the reference image is reconstructed under the second reconstruction rule from the second projection data, and wherein the first reconstruction rule specifies a first mixing of the second projection data according to the first energy band and the second energy band, and wherein the second reconstruction rule specifies a second mixing of the second projection data according to the first energy band and the second energy band.

9. Computed tomography system (20) for providing a medical image of a patient (P), comprising:
- a computed tomography apparatus (21), wherein the computed tomography apparatus (21) has at least one X-ray source (23) and at least one X-ray detector (24); and
- a processing unit (22),
wherein the computed tomography system (20) is designed to perform a method according to one of claims 1 to 8.

10. Computed tomography system (20) according to claim 9, wherein the computed tomography apparatus (21) has two X-ray sources (23) and two X-ray detectors (24).

11. Computer program product having program code means that can be loaded into a processing unit (22) in order to perform a method according to one of claims 1 to 8 when the program code means are executed in the processing unit (22).

## Revendications

1. Procédé pour disposer d'une image médicale d'un patient (P) prise au moyen d'un tomodensitomètre (21) assisté par ordinateur, comprenant les stades suivants :
- on saisit des premières données de projection d'une première région à mesurer au moyen du tomodensitomètre (21) assisté par ordinateur, dans lequel, à partir des premières données de projection, on reconstruit suivant une respiration du patient une image du patient corrélée à la respiration,
- on saisit des deuxièmes données de projection d'une deuxième région à mesurer au moyen du tomodensitomètre (21) assisté par ordinateur, dans lequel, à partir des deuxièmes données de projection, on reconstruit, suivant une première prescription de reconstruction, au moins une image fonctionnelle du patient et dans lequel la première région à mesurer et la deuxième région à mesurer se chevauchent au moins en partie,
- on enregistre une image de référence avec la au moins une image du patient corrélée à la respiration, un modèle de déformation étant produit,
- on applique le modèle de déformation à la au moins une image fonctionnelle du patient,
- on combine la au moins une image fonctionnelle déformée du patient à la au moins une image du patient corrélée à la respiration, l'image médicale du patient étant produite, et
- on met à disposition l'image médicale du patient, **caractérisé**
**en ce que** l'on reconstruit l'image de référence, suivant une deuxième prescription de reconstruction, à partir des deuxièmes données de projection, dans lequel la première prescription de reconstruction est optimisée du point de vue d'un contraste fonctionnel et dans lequel la deuxième prescription de reconstruction est optimisée du point de vue d'un contraste anatomique.

2. Procédé suivant la revendication 1, dans lequel on combine la au moins une image fonctionnelle du patient déformée à la au moins une image du patient corrélée à la respiration de manière à ce que l'image médicale du patient ait une image de soustraction du patient, qui a un enrichissement en agent de contraste du patient.

3. Procédé suivant l'une des revendications précédentes, dans lequel, après la saisie des premières données de projection et avant la saisie des deuxièmes données de projection, on injecte un bolus d'agent de contraste dans le patient.

4. Procédé suivant l'une des revendications précédentes, dans lequel les deuxièmes données de projection et l'image de référence, qui en est reconstruite, ont plusieurs états d'un enrichissement en agent de contraste dans le patient et dans lequel on enregistre l'image de référence en fonction des plusieurs états de l'enrichissement en agent de contraste avec la au moins une image du patient corrélée à la respiration.

5. Procédé suivant l'une des revendications précédentes, dans lequel, à partir des premières données de projection, on reconstruit la au moins une image corrélée à la respiration pour plusieurs états respiratoires de la respiration du patient et dans lequel par l'enregistrement de l'image de référence avec la au moins une image corrélée à la respiration respective pour les plusieurs états respiratoires de la respiration du patient, on produit plusieurs modèles de déformation.

6. Procédé suivant la revendication 5, dans lequel, par l'application des plusieurs modèles de déformation à la au moins une image fonctionnelle du patient et par la combinaison de la au moins une image fonctionnelle déformée pour les plusieurs états respiratoires du patient à la au moins une image du patient corrélée à la respiration, on produit l'image médicale pour les plusieurs états respiratoires de la respiration du patient.

7. Procédé suivant l'une des revendications précédentes, dans lequel on saisit les deuxièmes données de projection au moyen d'une séquence de mesure spectrale donnant une image dans le tomodensitomètre (21) assisté par ordinateur et elles ont un premier domaine d'énergie ainsi qu'un deuxième domaine d'énergie.

8. Procédé suivant la revendication 7, dans lequel on reconstruit l'image de référence, suivant la deuxième prescription de reconstruction, à partir des deuxièmes données de projection et dans lequel la première prescription de reconstruction décrit un premier mélange des deuxièmes données de projection suivant le premier domaine d'énergie et le deuxième domaine d'énergie et dans lequel la deuxième prescription de reconstruction décrit un deuxième mélange des deuxièmes données de projection suivant le premier domaine d'énergie et le deuxième domaine d'énergie.

9. Installation (20) de tomodensitométrie assistée par ordinateur pour disposer d'une image médicale d'un patient (P), comportant
- un tomodensitomètre (21) assisté par ordinateur, le tomodensitomètre (21) assisté par ordinateur ayant d'au moins une source (23) de rayons X et au moins un détecteur (24) de rayons X, et
- une unité (22) informatique,
l'installation (20) de tomodensitométrie assistée par ordinateur étant constituée pour effectuer un procédé suivant l'une revendications 1 à 8.

10. Installation (20) de tomodensitométrie assistée par ordinateur suivant la revendication 9, dans laquelle le tomodensitomètre (21) assisté par ordinateur a deux sources (23) de rayons X et deux détecteurs (24) de rayons X.

11. Produit de programme d'ordinateur, ayant des moyens de code de programme, qui peuvent être chargés dans une unité (22) informatique, pour effectuer un procédé suivant l'une des revendications 1 à 8, lorsque les moyens de code de programme sont réalisés dans l'unité (22) informatique.
